# FASCICULE DE BREVET EUROPEEN

(11) **EP 1 048 288 B1**
(45) Date de publication et mention de la délivrance du brevet: **27.06.2007**
(21) Numéro de dépôt: 00401069.0
(22) Date de dépôt: 18.04.2000
(51) Int. Cl.: A61Q 5/00, A61Q 5/02, A61Q 5/12, A61K 8/41, A61K 8/43, A61K 8/49

(54) **Utilisation de composés antifongiques halogénés et de composés antibactériens cationiques pour traiter les pellicules**
Verwendung von Halogenierten Antimykotischen- und Kationischen Antibakteriellen-Agentien zur Behandlung von Schuppen
Use of halogenated antimycotic- and cationic antibacterial-compounds for the treatment of dandruff

(30) Priorité: 26.04.1999 FR 9905249
(43) Date de publication de la demande: 02.11.2000
(73) Titulaire: L'ORÉAL, 75008 Paris (FR)
(72) Inventeur: Saint-Leger, Didier, 92400 Courbevoie (FR)
(74) Mandataire: Dossmann, Gérard

(56) Documents cités:
- EP-A- 0 196 824
- EP-A- 0 338 850
- EP-A- 0 680 745
- EP-A- 0 843 002
- WO-A-93/07847
- FR-A- 2 275 194
- FR-A- 2 714 603
- US-A- 5 643 937
- US-A- 5 834 409

## Description

La présente invention est relative à l'utilisation d'au moins un composé antifongique halogéné et d'au moins un composé antibactérien cationique dans une composition cosmétique antipelliculaire ou pour la préparation d'une composition dermatologique destinée au traitement des rougeurs cutanées et/ou des désordres desquamatifs liés à Malassezia spp. Les désordres desquamatifs du cuir chevelu tels que les pellicules ou les dermites séborrhéiques sont liés à la présence d'une levure caractéristique appelée le Malassezia ovalis, levure anciennement dénommée Pityrosporum (P. ovale et P. orbiculare).

Par ailleurs, il est connu que cette levure est capable de changer de forme et de métabolisme. En particulier elle peut évoluer vers une forme filamenteuse (Malassezia furfur), responsable de rougeurs cutanées.

Le traitement usuel de toutes ces affections fait intervenir l'utilisation d'agents antifongiques dans un milieu, tel qu'un shampooing, un gel ou une lotion, qui est apte à répartir ces agents et à les déposer sur les téguments.

Le pouvoir antifongique de telles substances (climbazole, kétoconazole, octopirox, zinc pyridinethione, etc) est limité; de plus, la rémanence du pouvoir antifongique est faible. Ainsi, les traitements utilisant ces antifongiques sont d'efficacité très moyenne, voir faible.

La demande EP 0843002 décrit des compositions détergentes à base de tensio-actifs anioniques, d'un germicide et d'un agent antibactérien. Le pouvoir antifongique des compositions décrites reste faible.

La demanderesse a donc cherché à résoudre ces problèmes et à obtenir des traitements plus efficaces.

Elle a découvert, de façon surprenante, qu'en associant spécifiquement certains composés antifongiques halogénés avec certains composés antibactériens cationiques, il était possible d'obtenir des traitements cosmétiques ou dermatologiques bien plus efficaces que ceux de l'art antérieur. En effet, il a été constaté que les associations spécifiques de l'invention présentaient une activité antifongique renforcée et durable.

De plus, il apparaît de façon surprenante que les agents antibactériens cationiques sélectionnés n'inhibent pas l'action des composés antifongiques halogénés sélectionnés mais au contraire, que ces associations présentent une activité antifongique renforcée dans l'amplitude et dans le temps.

Ainsi, dans les associations spécifiques de l'invention, les composés antifongiques ou antibactériens peuvent être utilisés à de plus faibles concentrations, tout en restant aussi, sinon plus efficaces que s'ils étaient utilisés seuls.

Il a été également constaté que les associations de l'invention présentaient une bonne solubilité et une bonne tolérance cutanée.

D'autres objets apparaîtront à la lumière de la description et des exemples qui suivent.

La présente invention concerne donc l'utilisation d'au moins un composé antifongique halogéné et d'au moins un composé antibactérien cationique dans ou pour la préparation d'une composition cosmétique ou dermatologique de pH supérieur ou égal à 7 destinée au traitement des rougeurs cutanées et/ou des désordres desquamatifs liés à Malassezia spp, à l'exception de l'association de la chlorhexidine comme composé antibactérien cationique avec le kétoconazole comme composé antifongique halogéné.

Un autre objet de l'invention consiste en un procédé de traitement cosmétique pour l'élimination des pellicules des cheveux et du cuir chevelu mettant en oeuvre ces compositions.

La présente invention a essentiellement pour objet l'utilisation d'au moins un composé antibactérien cationique (a):
- de formule (I) suivante: 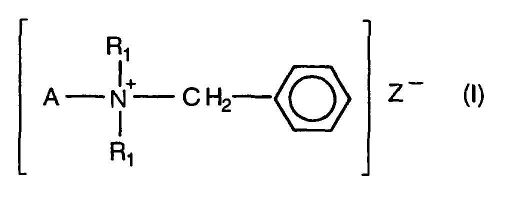 dans laquelle A est un groupement alkyl ou alkényl ayant de 8 à 18 atomes de carbone ou un groupe de formule suivante: 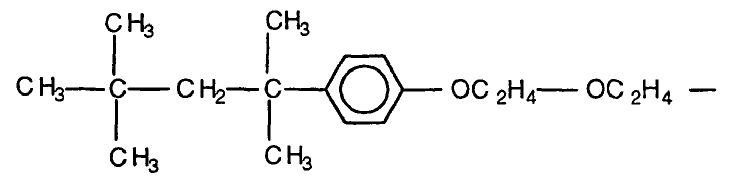 et dans laquelle R₁ et R₂ désignent indépendamment l'un de l'autre un radical alkyle en C₁-C₄; de préférence R₁ et R₂ désignent tous deux un radical méthyle;
   Z est un atome d'halogène, un reste anionique d'un acide aminé, d'un acide gras, ou un phosphate, phosphonate, sulfonate ou sulfate ayant un groupement alkyl ou alkényl linéaire ou branché de 1 à 30 atomes de carbone, ou un oligomère anionique ou un polymère ayant un acide styrène sulfonique avec un degré de polymérisation d'au moins 3 ou contenant un condensé d'un composé polycyclique aromatique sulfonaté, qui peut avoir un groupement hydrocarboné comme substituant, avec de la formaline,
- ou de formule (II) suivante: 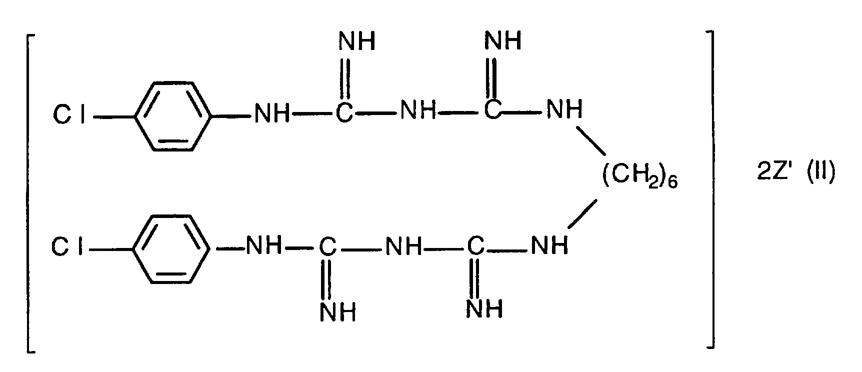 dans laquelle Z' est un anion monovalent tel que les anions dérivés d'acides physiologiquement acceptables et en particulier l'acide gluconique, l'acide acétique ou l'acide chlorhydrique;
   avec au moins un composé antifongique halogéné (b):
- de formule (III) suivante: 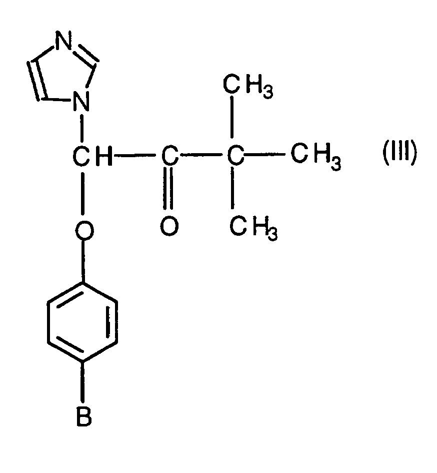 dans laquelle B représente un atome d'halogène ,
   ou un de ses sels d'addition d'acides physiologiquement acceptables;
- ou de formule (IV) suivante: 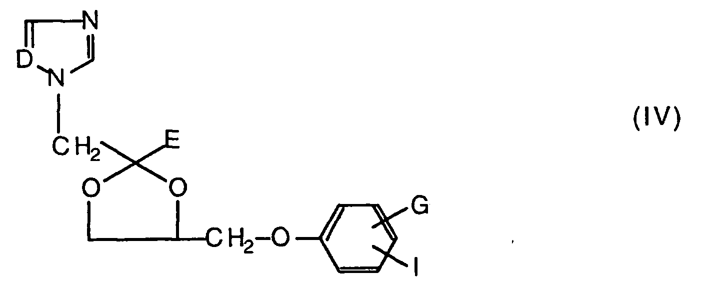
dans laquelle :
D représente CH ou N;
E représente un groupement phényle pouvant être substitué par 1 à 3 atomes d'halogène, groupements alkyle ou alcoxy;
G désigne :
   a) un groupement -N=C=S; ou
   b) un radical de formule 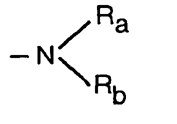 où Rₐ et R_{b}, indépendamment l'un de l'autre, désigne un atome d'hydrogène ou un groupement alkyle; ou
   c) un radical de formule: 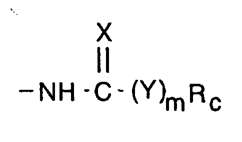 où X désigne un atome O ou S, Y désigne O ou NH, m désigne un nombre entier O ou 1, et R_{c} désigne hydrogène, un groupement alkyle, alkyle mono- ou dihalogéné, phényle pouvant être substitué par 1 à 2 atomes d'halogène, groupements alkyle ou alcoxy; ou
   d) un radical ayant pour formule 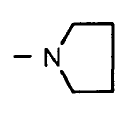
ou 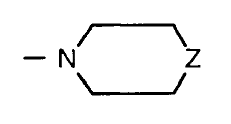 où Z désigne CH₂, O ou N-R₁₂, où R₁₂ désigne un atome d'hydrogène, un groupement alkyle, un groupement mono ou polyhydroxyalkyle, alcoxy, alcoxy alkyle, acyle, alkylsulfonyle, phénylméthylsulfonyle, alcoxycarbonyle, acyloxy, alcoxycarbonyl alkyle, phénoxycarbonyle, aminocarbonyle, mono ou di-alkyle aminocarbonyle, aminocarbonylalkyle, alkyle aminocarbonylalkyle, alkyle aminothioxoalkyle, alkylthioxoalkyle, aryle, arylalkyle ou benzoyle pouvant être substitué par 1 à 2 atomes d'halogène, un groupement alkyle inférieur ou alcoxy inférieur; et I désigne hydrogène ou un groupement nitro,
ou un de ses sels d'addition d'acides physiologiquement acceptables;
dans ou pour la préparation d'une composition cosmétique de pH supérieur ou égal à 7 destinée au traitement des rougeurs cutanées et/ou des désordres desquamatifs liés à Malassezia spp, à l'exception de l'association de la chlorhexidine comme composé antibactérien cationique avec le kétoconazole comme composé antifongique halogéné.

La composition définie ci-dessus est préférentiellement destinée au traitement antipelliculaire des cheveux et du cuir chevelu. En effet, il a été constaté que l'utilisation des compositions de la présente invention permettait d'améliorer l'esthétique des cheveux et du cuir chevelu.

L'utilisation des compositions de l'invention est également destinée à traiter d'autres rougeurs cutanées et/ou désordres desquamatifs liés à Malassezia spp, qui englobent notamment les désordres desquamatifs du cuir chevelu tels que les dermites séborrhéiques liées à la présence d'une levure caractéristique appelée Malassezia ovalis et/ou les rougeurs cutanées liés à Malassezia furfur.

Dans le cadre de la présente invention :

Un atome d'halogène désigne un atome de fluor, de chlore, de bromure ou d'iode et préférentiellement un atome de chlore.

Les groupements alkyle désignent des groupements linéaires ou ramifiés de 1 à 20 atomes de carbone comme par exemple des groupements alkyle inférieur, des groupements octyle, nonyle, décyle, dodécyle ou pentadécyle.

Les groupements alkyle désignent préférentiellement des groupements alkyls inférieurs linéaires ou ramifiés de 1 à 4 atomes de carbone, par exemple le groupement méthyle, éthyle, propyle, isopropyle, n-propyle, butyle, n-butyle ou tert.-butyle. Les groupements alkényles désigne de préférence un groupement ayant de 2 à 5 atomes de carbone.

Les groupements aryle peuvent désigner phényle ou naphtyle et de préférence phényle. Parmi les groupements alkylaryle, on peut notamment citer le groupement benzyle, phénéthyle ou naphthylméthyle.

Préférentiellement, le composé de formule (I), correspond au au chlorure de benzéthonium et plus préférentiellement au chlorure de benzalkonium.

Dans une forme de réalisation préférée de l'invention, le composé de formule (II) correspond à la chlorhexidine (sous forme de sel).

Préférentiellement le composé de formule (III) correspond au climbazole.

Le composé de formule (IV) peut être choisi parmi:
1-acétyl-4-{4-[2-(2,4-dichlorophényl)-2-(1H-imidazol-1-ylméthyl)-1,3-dioxolan-4-ylméthoxy]phényl}-pipérazine ;
4-{4-[2-(2,4-dichlorophényl)-2-(1H-imidazol-1-ylméthyl)-1,3-dioxolan-4-ylméthoxy]phényl}-morpholine ;
N-{4-[2-(2,4-dichlorophényl)-2-(1H-imidazol-1-ylméthyl)-1,3-dioxolan-4-ylméthoxy]phényl}-formamide ;
4- {4-[2-(2,4-dichlorophényl)-2-(1H-imidazol-1-ylméthyl)-1,3-dioxolan-4-ylméthoxy]phényl}-N-méthyl-1-pipérazinecarboxamide ;
éthyl 4-{4-[2-(2,4-dichlorophényl)-2-(1H-imidazol-1-ylméthyl)-1,3-dioxolan-4-ylméthoxy]phényl}-1-pipérazinecarboxylate ;
méthyl 4-{4-[2-(2,4-dichlorophényl-2-(1H-imidazol-1-ylméthyl)-1,3-dioxolan-4-ylméthoxy]phényl}-1-pipérazinecarboxylate ;
cis-1-acétyl-4-{4-[2-(2,4-dichlorophényl)-2-(1H-imidazol-1-yl-méthyl)-1,3-dioxolan-4-ylméthoxy]-phényl}pipérazine ;
cis-1{4-[2-(2,4-dichlorophényl)-2-1H-1,2,4-triazol-1-méthyl)-1,3-dioxolan-4-ylméthoxy]-phényl}-4-(1-méthyléthyl)-pipérazine ;
et leurs sels d'addition d'acides physiologiquement acceptables.

Plus particulièrement, le composé 1-acétyl-4-{4-[2-(2,4-dichlorophényl)-2-(1H-imidazol-1-ylméthyl)-1,3-dioxolan-4-ylméthoxy]phényl}-pipérazine, encore appelé kétoconazole est particulièrement préféré.

Les composés ci-dessus peuvent aussi être utilisés sous forme de leurs sels d'addition d'acides physiologiquement acceptables choisis parmi les sels d'acide sulfurique, nitrique, thiocyanique, chlorhydrique, bromhydrique, iodhydrique, phosphorique, acétique, benzoïque, salicylique, glycolique, acéturique, succinique, nicotinique, tartrique, maléique, palmitique, méthane sulfonique, propanoïque, hydroxyacétique, 2-hydroxypropanoïque, 2-oxopropanoïque, éthanedioïque, propanedioïque, 1,4-butane-dioïque, 2-hydroxy-1,4-butanedioïque, 2,3-dihydroxy-1,4-butanedioïque, 2-hydroxy-1,2,3-propanetricarboxylique, 3-phényl-2-propènoïque, α-hydroxybenzèneacétique, éthanesulfonique, 2-hydroxyéthanesulfonique, 4-méthylbenzènesulfonique, 2-hydroxy-benzoïque, 4-amino-2-hydroxybenzoïque, 2-phénoxybenzoïque, 2-acétyloxybenzoïque, picrique, lactique et d'aminoacides.

Le composé (a) et le composé (b) peuvent être présents dans des proportions comprises entre 0,001% et 10% en poids par rapport au poids total de la composition et préférentiellement dans des proportions comprises entre 0,01% et 5% en poids par rapport au poids total de la composition.

Le rapport pondéral du composé (a) au composé (b) peut varier de 0,1 à 10 et préférentiellement de 0,3 à 3.

Le pH des compositions utilsées dans l'invention est dans une forme de réalisation préférée de l'invention est compris entre 7,2 et 9 et préferentiellement entre 7,5 et 8,5. En effet, il a été constaté que les compositions utilisées dans l'invention étaient particulièrement efficaces dans ces gammes de pH.

Les compositions selon l'invention peuvent se présenter sous forme de liquides plus ou moins épaissis, de gels, de crèmes ou de mousses aérosols.

Les compositions conformes à l'invention peuvent éventuellement contenir en outre divers additifs qui n'altèrent pas la stabilité ni le pouvoir antifongique des compositions de l'invention, tels que des agents tensio-actifs anioniques, amphotères ou zwittérioniques, non-ioniques, des agents de mises en suspension, des polymères anioniques, non-ioniques cationiques ou amphotères, des protéines, des huiles, des cires, des résines et/ou des gommes de silicone, des agents acidifiants ou alcalinisants, des agents conservateurs, des parfums ou autres adjuvants couramment utilisés en cosmétique.

Dans une forme préférée de réalisation de l'invention, les compositions contiennent au moins un tensio-actif non-ionique du type polyglycérolé ou du type alkylpolyglycoside et un agent de mise en suspension choisi parmi les dérivés de cellulose anioniques et les biopolysaccharides.

Les alkylpolylycosides sont de préférence choisis parmi les produits vendus par la Société HENKEL sous la dénomination APG, tels que les produits APG 300, APG 350, APG 500, APG 550, APG 625, APG base 10-12; les produits vendus par la Société SEPPIC sous les dénominations TRITON CG 110 (ou ORAMIX CG 110) et TRITON CG 312 (ou ORAMIX NS 10); ceux vendu par la Société BASF sous la dénomination LUTENSOL GD 70.

Les biopolysaccharides utilisés sont des produits comportant dans leur structure des unités glucose, mannose, acide glucuronique ou galacturonique ou D-glucopyranose ou galactose.

Les dérivés de cellulose anioniques sont préférentiellement la carboxyméthylcellulose de sodium et les biopolysaccharides, les gommes de xanthane ou de scléroglucane.

Les compositions selon l'invention peuvent renfermer le ou les tensioactifs, notamment lorsqu'elles sont utilisées comme compositions lavantes, telles que des shampooings, dans des proportions comprises de préférence entre 5 et 50% en poids par rapport au poids total de la composition et plus particulièrement entre 8 et 30% en poids.

Les agents de mise en suspension tels que définis ci-dessus, peuvent être présents dans les compositions de l'invention dans des proportions comprises de préférence entre 0,2 et 5% en poids par rapport au poids total de la composition et plus particulièrement entre 0,5 et 3% en poids.

Les compositions selon l'invention peuvent également contenir d'autres agents anti-bactériens que ceux décrits précédemment tels que la chloramine T, la chloramine B, le 1,3-dibromo 5,5-diméthylhydantoïne, le 1,3-dichloro 5,5-diméthylhydantoïne, le 3-bromo 1-chloro 5,5-diméthylhydantoïne ou la N-chlorosuccinimide.

Elles peuvent aussi contenir d'autres agents antifongiques que ceux décrits ci-dessus, comme par exemple le sulfure de sélenium, l'octopirox ou la zincpyridinethione.

Un objet de l'invention est aussi un procédé de traitement cosmétique pour l'élimination des pellicules, consistant en l'application d'une composition telle que définie ci-dessus et que l'on fait suivre éventuellement d'un rinçage.

Dans une forme de réalisation préférée de l'invention, les compositions selon l'invention sont utilisées comme shampooings pour le lavage et le traitement des cheveux et du cuir chevelu et ils sont appliqués, dans ce cas-là, sur des cheveux humides ou secs dans des quantités efficaces pour les laver, cette application étant suivie d'un rinçage.

Les exemples qui suivent illustrent la présente invention sans toutefois présenter un caractère limitatif.

### Exemple I : LOTION ANTIPELLICULAIRE

| | | | | |
|---|---|---|---|---|
| Climbazole | | | 0,3 | g |
| Chlorhexidine | | | 0,4 | g |
| Propylène glycol | | | 20 | g |
| Ethanol 95° | | | 30 | g |
| Triéthanolamine | qs | pH | 7,5 | |
| Eau | qsp | | 100 | g |

Cette lotion est appliquée quotidiennement à raison de 6 ml sur le cuir chevelu et ce pendant 1 semaine. On constate alors une chute rapide des sensations de prurit suivie par une nette amélioration de l'état pelliculaire.

### Exemple II: SHAMPOOING ANTIPELLICULAIRE

| | | | | |
|---|---|---|---|---|
| Climbazole | | | 0,5 | g |
| Chlorure de Benzalkonium | | | 0,5 | g |
| Polyglyceryl 3-hydroxylaurylether | | | 26 | g M.A. |
| Hydroxy propyl cellulose vendue | | | | |
| sous la dénomination de Klucell G par la | | | | |
| Sté HERCULES | | | 2 | g |
| Conservateur | | | qs | |
| Ethanol 95° | | | 50 | g |
| Triéthanolamine | qs | pH | 7,5 | |
| Eau | qsp | | 100 | g |

Ce shampooing est utilisé quotidiennement à raison de 10 g par chevelure avec un temps de pose de l'ordre d'une minute et ceci pendant une période de 2 semaines. On observe alors une chute rapide des sensations de prurit et une nette amélioration de l'état pelliculaire.

### Exemple III : LOTION ANTIPELLICULAIRE

| | | | | |
|---|---|---|---|---|
| Kétoconazole | | | 0,1 | g |
| Chlorure de Benzalkonium | | | 0,3 | g |
| Propylène glycol | | | 20 | g |
| Ethanol 95° | | | 30 | g |
| Soude | qs | pH | 8 | |
| Eau | qsp | | 100 | g |

Cette lotion est appliquée quotidiennement à raison de 8 ml sur le cuir chevelu et ce pendant 2 semaines. On constate alors une chute rapide des sensations de prurit suivie par une nette amélioration de l'état pelliculaire.

### Exemple IV : SHAMPOOING ANTIPELLICULAIRE

| | | | | |
|---|---|---|---|---|
| Climbazole | | | 0,5 | g |
| D.Gluconate de chlorhexidine | | | 0,75 | g |
| Alkylpolyglucoside vendu sous la | | | | |
| dénomination d'APG 300 par la | | | | |
| sté HENKEL | | | 10 | g MA |
| Hydroxy propyl cellulose vendue sous la | | | | |
| dénomination de Klucell G par la | | | | |
| sté HERCULES | | | 2 | g |
| Conservateur | | | qs | |
| Ethanol 95° | | | 50 | g |
| 2-Amino2-méthyl1-propanol | qs | pH | 8 | |
| Eau | qsp | | 100 | g |

Ce shampooing est utilisé quotidiennement à raison de 10 g par chevelure avec un temps de pose de l'ordre d'une minute et ceci pendant une période de 15 jours. On observe alors une chute rapide des sensations de prurit et une nette amélioration de l'état pelliculaire.

### Exemple V : SHAMPOOING ANTIPELLICULAIRE

| | | | | |
|---|---|---|---|---|
| Chlorure de Benzalkonium | | | 0,6 | g |
| Kétoconazole | | | 0,5 | g |
| Polyglyceryl 3-hydroxylaurylether | | | 26 | g M.A. |
| Hydroxy propyl cellulose vendue sous la | | | | |
| dénomination de Klucell G par la | | | | |
| Sté HERCULES | | | 2 | g |
| Conservateur | | | 50 | g |
| Triéthanolamine | qs | pH | 7,5 | |
| Eau | qsp | | 100 | g |

Ce shampooing est utilisé quotidiennement à raison de 10 g par chevelure avec un temps de pose de l'ordre d'une minute et ceci pendant une période de 2 semaines. On oberve alors une chute rapide des sensations de prurit et une nette amélioration de l'état pelliculaire.

### Exemple VI: Détermination de l'activité antifongique des associations des composés (a) et (b)

L'activité des associations des composés (a) et (b) a été déterminée par une méthode classique dite de CMI (Concentration Minimale Inhibitrice), la CMI correspondant à la concentration minimale à laquelle un produit donné inhibe la pousse d'une souche donnée dans des conditions définies. En l'occurrence, des souches de Pityrosporum ovale de l'Institut Pasteur référence CIP 1363.82 ont été utilisées.

200 µl de composé ou d'association ont été ajoutés à 1,8 ml de milieu de culture gélosé (milieu gélose de Sabouraud+ Tween 40 à 10g/l + mono oléate de glycérol à 2.5 g/l) en surfusion à 45°C. Des dilutions successives, en progression géométrique de raison 2, de la suspension obtenue ont été réalisées à l'aide du milieu de culture gélosé en surfusion dans les puits d'une plaque (Falcon, 24 puits). Après solidification du milieu, 4 µl de la suspension microbienne ont été déposés en surface à l'aide d'une micropipette.

Après 48 heures d'incubation à 30°C, la concentration du composé ou de l'association de composés à partir de laquelle la pousse du micro-organisme est complètement inhibée (absence de trouble du milieu) est ainsi déterminée.

Pour les composés étudiés, non associés à d'autres, à la concentration de CMI/2 et à plus forte raison à la CMI/4, la pousse est identique à celle du milieu témoin, milieu sans composé antifongique ou antibactérien.

La pousse est en revanche bloquée par les combinaisons suivantes:
- Climbazole à CMI/2 + Chlorure de Benzalkonium à CMI/2
- Kétoconazole à CMI/2 + Chlorure de Benzalkonium à CMI/2
- Climbazole à CMI/4 + gluconate de Chlorhexidine à CMI/2

Ces résultats montrent donc que ces associations de composés, à des doses ou leur activité propre est perdue, restent efficaces, voire présentent une activité antifongique renforcée.

## Revendications

1. Utilisation d'au moins un composé antibactérien cationique (a) :
• de formule (I) suivante: 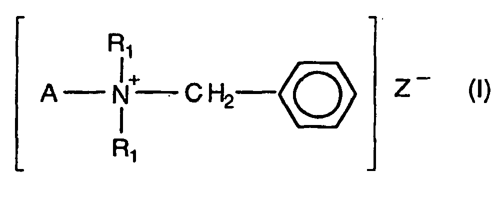 dans laquelle:
A est un groupement alkyl ou alkényl ayant de 8 à 18 atomes de carbone ou un groupe de formule suivante: 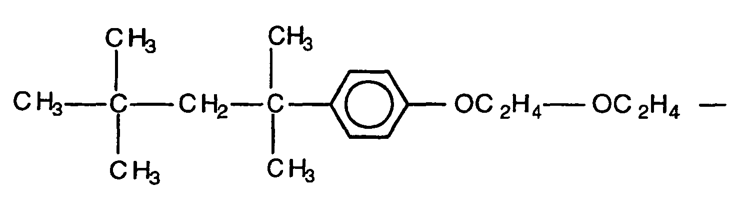
R₁ et R₂ désignent indépendamment l'un de l'autre un radical alkyle inférieur en C₁-C₄;
Z est un atome d'halogène, un reste anionique d'un acide aminé, d'un acide gras, ou un phosphate, phosphonate, sulfonate ou sulfate ayant un groupement alkyl ou alkenyl linéaire ou branché de 1 à 30 atomes de carbone, ou un oligomère anionique ou un polymère ayant un acide styrène sulfonique avec un degré de polymérisation d'au moins 3 ou contenant un condensé d'un composé polycyclique aromatique sulfonaté, qui peut avoir un groupement hydrocarboné comme substituant, avec de la formaline;
• ou de formule (II) suivante: 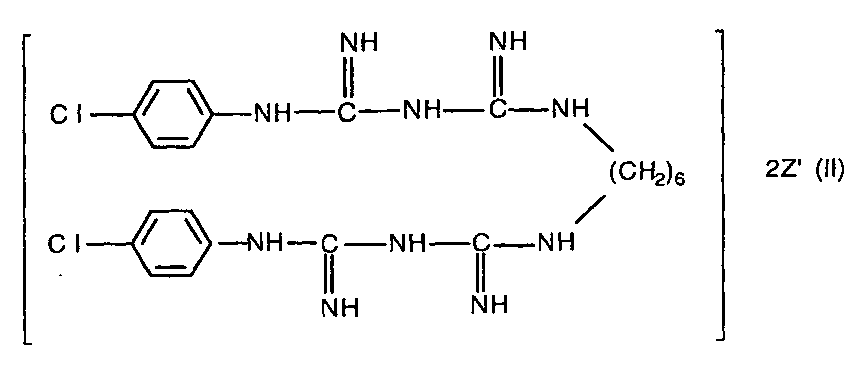 dans laquelle Z' est l'acide gluconique, l'acide acétique ou l'acide chlorhydrique,
avec au moins un composé antifongique halogéné (b):
• de formule (III) suivante: 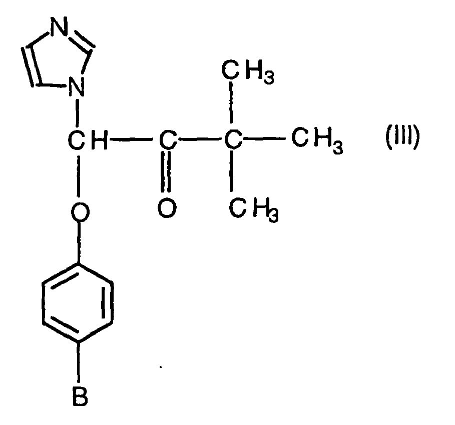 dans laquelle B représente un atome d'halogène;
• ou de formule (IV) suivante: 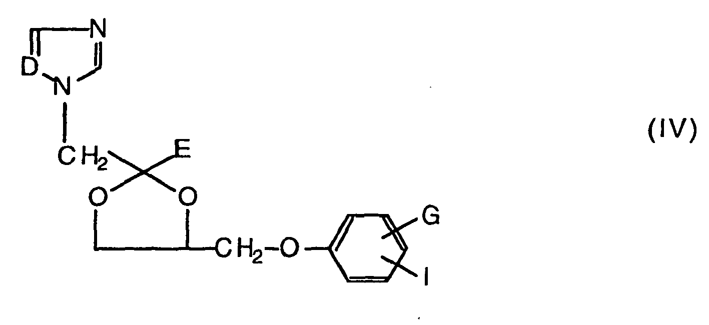 dans laquelle :
D représente CH ou N;
E représente un groupement phényle pouvant être substitué par 1 à 3 atomes d'halogène, groupements alkyle ou alcoxy;
G désigne :
a) un groupement -N=C=S; ou
b) un radical de formule 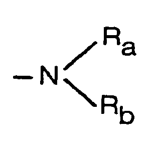 où Rₐ et R_{b}, indépendamment l'un de l'autre, désigne un atome d'hydrogène ou un groupement alkyle; ou
c) un radical de formule: 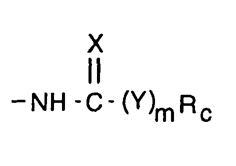 où X désigne un atome O ou S, Y désigne O ou NH, m désigne un nombre entier O ou 1, et R_{c} désigne hydrogène, un groupement alkyle, alkyle mono- ou dihalogéné, phényle pouvant être substitué par 1 à 2 atomes d'halogène, groupements alkyle ou alcoxy; ou
d) un radical ayant pour formule 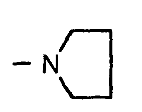 ou 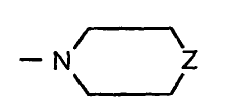 où Z désigne CH₂, O ou N-R₁₂, où R₁₂ désigne un atome d'hydrogène, un groupement alkyle, un groupement mono ou polyhydroxyalkyle, alcoxy, alcoxy alkyle, acyle, alkylsulfonyle, phénylméthylsulfonyle, alcoxycarbonyle, acyloxy, alcoxycarbonyl alkyle, phénoxycarbonyle, aminocarbonyle, mono ou di-alkyle aminocarbonyle, aminocarbonylalkyle, alkyle aminocarbonylalkyle, alkyle aminothioxoalkyle, alkylthioxoalkyle, aryle, arylalkyle ou benzoyle pouvant être substitué par 1 à 2 atomes d'halogène, un groupement alkyle inférieur ou alcoxy inférieur; et I désigne hydrogène ou un groupement nitro,
ou un de ses sels d'addition d'acides physiologiquement acceptables;
dans ou pour la préparation d'une composition cosmétique ou dermatologique de pH supérieur ou égal à 7 destinée au traitement antipelliculaire des cheveux et du cuir chevelu à l'exception de l'association de la chlorhexidine comme composé antibactérien cationique avec le kétoconazole comme composé antifongique halogéné.

2. Utilisation selon la revendication 1, **caractérisée en ce que** le composé de formule (I) correspond au chlorure de benzalkonium ou au chlorure de benzéthonium.

3. Utilisation selon la revendication 1, **caractérisée en ce que** le composé de formule (II) correspond à la chlorhexidine sous forme salifiée.

4. Utilisation selon l'une quelconque des revendications 1 à 3, **caractérisée en ce que** le composé de formule (III) correspond au climbazole.

5. Utilisation selon l'une quelconque des revendications 1 à 3, **caractérisée en ce que** le composé de formule (IV), est choisi parmi:
1-acétyl-4-{4-[2-(2,4-dichlorophényl)-2-(1H-imidazol-1-ylméthyl)-1,3-dioxolan-4-ylméthoxy]phényl}-pipérazine ;
4-{4-[2-(2,4-dichlorophényl)-2-(1H-imidazol-1-ylméthyl)-1,3-dioxolan-4-ylméthoxy]phényl}-morpholine ;
N-{4-[2-(2,4-dichlorophényl)-2-(1H-imidazol-1-ylméthyl)-1,3-dioxolan-4-ylméthoxy]phényl}-formamide ;
4-{4-[2-(2,4-dichlorophényl)-2-(1H-imidazol-1-ylméthyl)-1,3-dioxolan-4-ylméthoxy]phényl}-N-méthyl-1-pipérazinecarboxamide ;
éthyl 4-{4-[2-(2,4-dichlorophényl)-2-(1H-imidazol-1-ylméthyl)-1,3-dioxolan-4-ylméthoxy]phényl}-1-pipérazinecarboxylate ;
méthyl 4-{4-[2-(2,4-dichlorophényl-2-(1H-imidazol-1-ylméthyl)-1,3-dioxolan-4-ylméthoxy]phényl}-1-pipérazinecarboxylate ;
cis-1-acétyl-4-{4-[2-(2,4-dichlorophényl)-2-(1H-imidazol-1-yl-méthyl)-1,3-dioxolan-4-ylméthoxy]-phényl}pipérazine ;
cis-1{4-[2-(2,4-dichlorophényl)-2-1H-1,2,4-triazol-1-méthyl)-1,3-dioxolan-4-ylméthoxy]-phényl}-4-(1-méthyléthyl)-pipérazine ;
et leurs sels d'addition d'acides physiologiquement acceptables.

6. Utilisation selon la revendication 5, **caractérisée en ce que** le composé de formule (IV), correspond au 1-acétyl-4-{4-[2-(2,4-dichlorophényl)-2-(1H-imidazol-1-ylméthyl)-1,3-dioxolan-4-ylméthoxy]phényl}-pipérazine ou à l'un de ses sels d'addition d'acides physiologiquement acceptables.

7. Utilisation selon l'une quelconque des revendications 1 à 6, **caractérisée par le fait que** les sels d'addition d'acides physiologiquement acceptables sont choisis parmi les sels d'acide sulfurique, nitrique, thiocyanique, chlorhydrique, bromhydrique, iodhydrique, phosphorique, acétique, benzoïque, salicylique, glycolique, acéturique, succinique, nicotinique, tartrique, maléique, palmitique, méthane sulfonique, propanoïque, hydroxyacétique, 2-hydroxypropanoïque, 2-oxopropanoïque, éthanedioïque, propanedioïque, 1,4-butane-dioïque, 2-hydroxy-1,4-butanedioïque, 2,3-dihydroxy-1,4-butanedioïque, 2-hydroxy-1,2,3-propanetricarboxylique, 3-phényl-2-propènoïque, α-hydroxybenzèneacétique, éthanesulfonique, 2-hydroxyéthanesulfonique, 4-méthylbenzènesulfonique, 2-hydroxy-benzoïque, 4-amino-2-hydroxybenzoïque, 2-phénoxybenzoïque, 2-acétyloxybenzoïque, picrique, lactique et d'aminoacides.

8. Utilisation selon l'une quelconque des revendications 1 à 7, **caractérisée par le fait que** le composé (a) et le composé (b) sont présents dans des proportions comprises entre 0,001% et 10% en poids par rapport au poids total de la composition.

9. Utilisation selon l'une quelconque des revendications 1 à 8, **caractérisée par le fait que** le composé (a) et le composé (b) sont présents dans des proportions comprises entre 0,01% et 5% en poids par rapport au poids total de la composition.

10. Utilisation selon l'une quelconque des revendications 1 à 9, **caractérisée par le fait que** le rapport pondéral du composé (a) au composé (b) varie de 0,1 à 10.

11. Utilisation selon la revendications 10, **caractérisée par le fait que** le rapport pondéral du composé (a) au composé (b) varie de 0,3 à 3.

12. Utilisation selon l'une quelconque des revendications 1 à 11, **caractérisée par le fait que** le pH de la composition est compris entre 7,2 et 9, et préferrentiellement entre 7,5 et 8,5.

13. Utilisation selon l'une quelconque des revendications 1 à 12, **caractérisée par le fait que** la composition se présente sous forme de liquide plus ou moins épaissi, de gel, de crème ou de mousse aérosol.

14. Utilisation selon l'une quelconque des revendications 1 à 13, **caractérisée par le fait que** la composition contient au moins un tensioactif anionique, amphotère, zwittérionique ou cationique.

15. Utilisation selon l'une quelconque des revendications 1 à 14, **caractérisée par le fait que** la composition contient au moins un tensioactif non-ionique.

16. Utilisation selon la revendication 15, **caractérisée par le fait que** la composition contient au moins un tensio-actif non-ionique du type polyglycérolé ou alkylpolyglycoside.

17. Utilisation selon la revendication 14 ou 16, **caractérisée par le fait que** l'agent tensio-actif non-ionique est présent dans des concentrations comprises entre 5 et 50% en poids par rapport au poids total de la composition.

18. Utilisation selon l'une quelconque des revendications 1 à **caractérisée par le fait que** la composition contient des additifs choisis parmi des agents de mise en suspension, des polymères anioniques, cationiques, non-ioniques, amphotères, des protéines, des huiles, des cires, des gommes et/ou des résines de silicone, des agents acidifiants ou alcalinisants, des conservateurs, des antibactériens et des antifongiques autres que ceux définis dans la revendication 1, des parfums ou autres adjuvants habituellement utilisés en cosmétique.

19. Utilisation selon l'une quelconque des revendications 1 à 18, **caractérisée par le fait que** la composition se présente sous forme de shampooing pour le lavage des cheveux et le traitement du cuir chevelu.

20. Procédé de traitement cosmétique pour l'élimination des pellicules des cheveux et du cuir chevelu, **caractérisé par le fait que** l'on applique une composition telle que définie dans l'une quelconque des revendications 1 à 19, et que l'on fait suivre éventuellement d'un rinçage.

## Claims

1. Use of at least one cationic antibacterial compound (a):
• of formula (I) below: 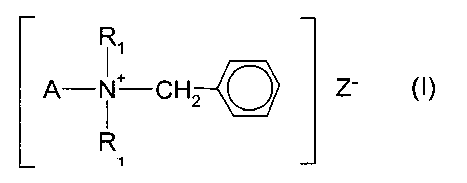 in which:
A is an alkyl or alkenyl group containing from 8 to 18 carbon atoms or a group having the following formula: 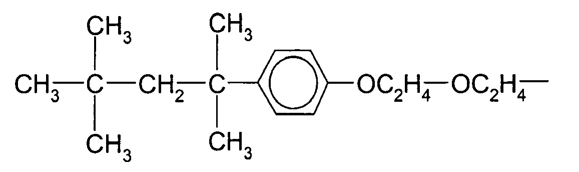
R₁ and R₂ denote, independently of each other, a C₁-C₄ lower alkyl radical;
Z is a halogen atom, an anionic residue of an amino acid or of a fatty acid, or a phosphate, phosphonate, sulfonate or sulfate containing a linear or branched alkyl or alkenyl group of 1 to 30 carbon atoms, or an anionic oligomer or a polymer containing a styrenesulfonic acid with a degree of polymerization of at least 3 or containing a condensate of a sulfonated aromatic polycyclic compound, which may contain a hydrocarbon-based group as substituent, with formalin;
• or of formula (II) below: 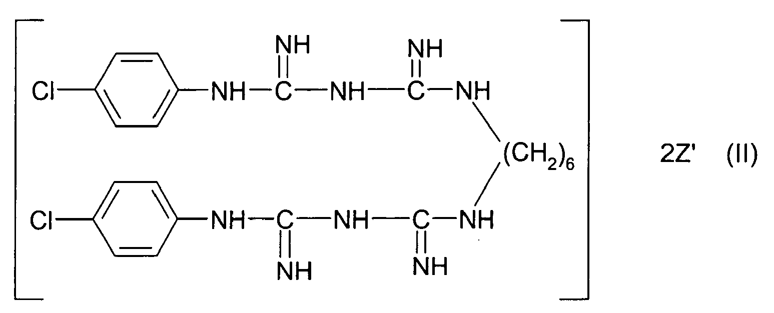 in which Z' is gluconic acid, acetic acid or hydrochloric acid;
with at least one halogenated antifungal compound (b):
• of formula (III) below: 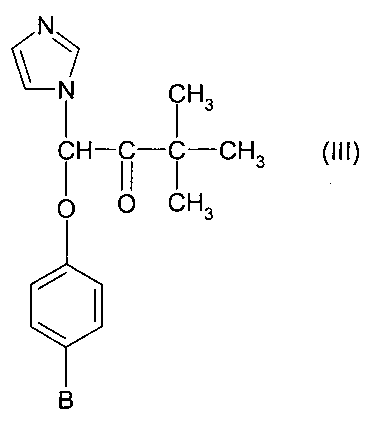 in which B represents a halogen atom
• or of formula (IV) below: 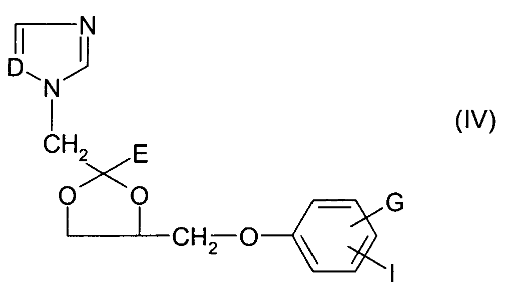 in which:
D represents CH or N;
E represents a phenyl group possibly substituted with 1 to 3 halogen atoms or alkyl or alkoxy groups;
G denotes:
a) a group -N=C=S; or
b) a radical of formula 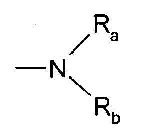 in which Rₐ and R_{b}, independently of each other, denote a hydrogen atom or an alkyl group; or
c) a radical of formula: 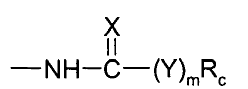 in which X denotes an O or S atom, Y denotes O or NH, m denotes an integer 0 or 1, and R_{c} denotes hydrogen, an alkyl, monohalo or dihalo alkyl, or phenyl group possibly substituted with 1 to 2 halogen atoms or alkyl or alkoxy groups; or
d) a radical having the formula 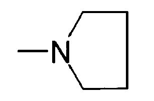 or 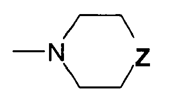
in which Z denotes CH₂, O or N-R₁₂, in which R₁₂ denotes a hydrogen atom, an alkyl group or a mono- or polyhydroxyalkyl, alkoxy, alkoxyalkyl, acyl, alkylsulfonyl, phenylmethylsulfonyl, alkoxycarbonyl, acyloxy, alkoxycarbonylalkyl, phenoxycarbonyl, aminocarbonyl, mono- or dialkylaminocarbonyl, aminocarbonylalkyl, alkyl aminocarbonylalkyl, alkyl aminothioxoalkyl, alkylthioxoalkyl, aryl, arylalkyl or benzoyl group possibly substituted with 1 to 2 halogen atoms, or a lower alkyl or lower alkoxy group; and
I denotes hydrogen or a nitro group,
or a physiologically acceptable acid-addition salt thereof;
in or for the preparation of a cosmetic or dermatological composition with a pH of greater than or equal to 7, for the antidandruff treatment of the hair and the scalp, with the exception of the combination of chlorhexidine as cationic antibacterial compound with ketoconazole as halogenated antifungal compound.

2. Use according to Claim 1, **characterized in that** the compound of formula (I) corresponds to benzalkonium chloride or benzethonium chloride.

3. Use according to Claim 1, **characterized in that** the compound of formula (II) corresponds to chlorhexidine in salified form.

4. Use according to any one of Claims 1 to 3,
**characterized in that** the compound of formula (III) corresponds to climbazole.

5. Use according to any one of Claims 1 to 3, **characterized in that** the compound of formula (IV) is chosen from:
1-acetyl-4-{4-[2-(2,4-dichlorophenyl)-2-(1H-imidazol-1-ylmethyl)-1,3-dioxolan-4-ylmethoxy]phenyl}piperazine;
4-{4-[2-(2,4-dichlorophenyl)-2-(1H-imidazol-1-yl-methyl)-1,3-dioxolan-4-ylmethoxy]phenyl}morpholine;
N-{4-[2-(2,4-dichlorophenyl)-2-(1H-imidazol-1-yl-methyl)-1,3-dioxolan-4-ylmethoxy]phenyl}formamide;
4-{4-[2-(2,4-dichlorophenyl)-2-(1H-imidazol-1-yl-methyl)-1,3-dioxolan-4-ylmethoxy]phenyl}-N-methyl-1-piperazinecarboxamide;
ethyl 4-{4-[2-(2,4-dichlorophenyl)-2-(1H-imidazol-1-yl-methyl)-1,3-dioxolan-4-ylmethoxy]phenyl}-1-piperazinecarboxylate;
methyl 4-{4-[2-(2,4-dichlorophenyl-2-(1H-imidazol-1-yl-methyl)-1,3-dioxolan-4-ylmethoxy]phenyl}-1-piperazinecarboxylate;
cis-1-acetyl-4-{4-[2-(2,4-dichlorophenyl)-2-(1H-imidazol-1-ylmethyl)-1,3-dioxolan-4-ylmethoxy]phenyl}-piperazine;
cis-1-{4-[2-(2,4-dichlorophenyl)-2-(1H-1,2,4-triazol-1-methyl)-1,3-dioxolan-4-ylmethoxy]phenyl}-4-(1-methylethyl)piperazine;
and the physiologically acceptable acid-addition salts thereof.

6. Use according to Claim 5, **characterized in that** the compound of formula (IV) corresponds to 1-acetyl-4-{4-[2-(2,4-dichlorophenyl)-2-(1H-imidazol-1-ylmethyl)-1,3-dioxolan-4-ylmethoxy]phenyl}piperazine or a physiologically acceptable acid-addition salt thereof.

7. Use according to any one of Claims 1 to 6, **characterized in that** the physiologically acceptable acid-addition salts are chosen from the salts of sulfuric acid, nitric acid, thiocyanic acid, hydrochloric acid, hydrobromic acid, hydriodic acid, phosphoric acid, acetic acid, benzoic acid, salicylic acid, glycolic acid, aceturic acid, succinic acid, nicotinic acid, tartaric acid, maleic acid, palmitic acid, methanesulfonic acid, propanoic acid, hydroxyacetic acid, 2-hydroxypropanoic acid, 2-oxopropanoic acid, ethanedioic acid, propanedioic acid, 1,4-butanedioic acid, 2-hydroxy-1,4-butanedioic acid, 2,3-dihydroxy-1,4-butanedioic acid, 2-hydroxy-1,2,3-propanetricarboxylic acid, 3-phenyl-2-propenoic acid, α-hydroxybenzeneacetic acid, ethanesulfonic acid, 2-hydroxyethanesulfonic acid, 4-methylbenzenesulfonic acid, 2-hydroxybenzoic acid, 4-amino-2-hydroxybenzoic acid, 2-phenoxybenzoic acid, 2-acetyloxybenzoic acid, picric acid, lactic acid and amino acids.

8. Use according to any one of Claims 1 to 7, **characterized in that** compound (a) and compound (b) are present in proportions of between 0.001% and 10% by weight relative to the total weight of the composition.

9. Use according to any one of Claims 1 to 8, **characterized in that** compound (a) and compound (b) are present in proportions of between 0.01% and 5% by weight relative to the total weight of the composition.

10. Use according to any one of Claims 1 to 9, **characterized in that** the weight ratio of compound (a) to compound (b) ranges from 0.1 to 10.

11. Use according to Claim 10, **characterized in that** the weight ratio of compound (a) to compound (b) ranges from 0.3 to 3.

12. Use according to any one of Claims 1 to 11, **characterized in that** the pH of the composition is between 7.2 and 9 and preferentially between 7.5 and 8.5.

13. Use according to any one of Claims 1 to 12, **characterized in that** the composition is in the form of a more or less thickened liquid, a gel, a cream or an aerosol foam.

14. Use according to any one of Claims 1 to 13, **characterized in that** the composition contains at least one anionic, amphoteric, zwitterionic or cationic surfactant.

15. Use according to any one of Claims 1 to 14, **characterized in that** the composition contains at least one nonionic surfactant.

16. Use according to Claim 15, **characterized in that** the composition contains at least one nonionic surfactant of the polyglycerolated or alkylpolyglycoside type.

17. Use according to Claim 14 or 16, **characterized in that** the nonionic surfactant is present in concentrations of between 5% and 50% by weight relative to the total weight of the composition.

18. Use according to any one of Claims 1 to 17, **characterized in that** the composition contains additives chosen from suspension agents, anionic, cationic, nonionic or amphoteric polymers, proteins, oils, waxes, silicone gums and/or resins, acidifying or basifying agents, preserving agents, antibacterial agents and antifungal agents other than those defined in Claim 1, fragrances or other adjuvants usually used in cosmetics.

19. Use according to any one of Claims 1 to 18, **characterized in that** the composition is in the form of a shampoo for washing the hair and treating the scalp.

20. Cosmetic treatment process for removing dandruff from the hair and the scalp, **characterized in that** a composition as defined in any one of Claims 1 to 19 is applied, and is optionally followed by rinsing.

## Patentansprüche

1. Verwendung mindestens einer kationischen antibakteriellen Verbindung (a):
• der folgenden Formel (I): 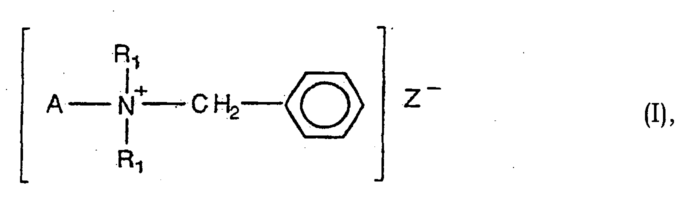 worin bedeuten:
A eine Alkyl- oder Alkenylgruppe mit 8 bis 18 Kohlenstoffatomen oder eine Gruppe der folgenden Formel: 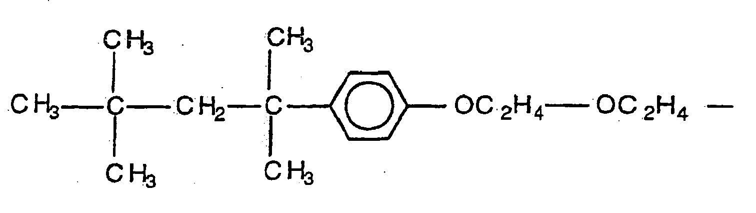
R₁ und R₂ unabhängig voneinander eine niedere C₁₋₄-Alkylgruppe;
Z ein Halogenatom, einen anionischen Rest einer Aminosäure, einer Fettsäure, oder ein Phosphat, Phosphonat, Sulfonat oder Sulfat mit einer geradkettigen oder verzweigten Alkyl- oder Alkenylgruppe mit 1 bis 30 Kohlenstoffatomen, oder ein anionisches Oligomer oder ein Polymer, das eine Styrolsulfonsäure mit einem Polymerisationsgrad von mindestens 3 aufweist oder ein Kondensat einer aromatischen polycyclischen Sulfonatverbindung, die als Substituenten eine Kohlenwasserstoffgruppe aufweisen kann, mit Formalin enthält;
• oder der folgenden Formel (II) : 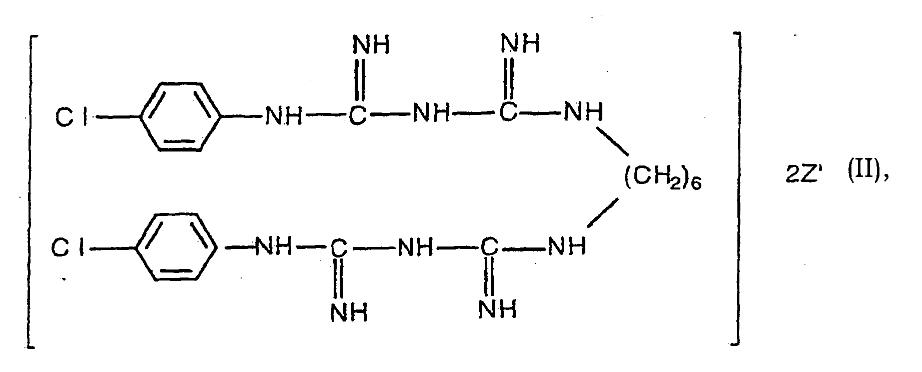 worin Z' Gluconsäure, Essigsäure oder Salzsäure bedeutet, und mindestens einer halogenierten antimykotischen Verbindung (b):
• der folgenden Formel (III): 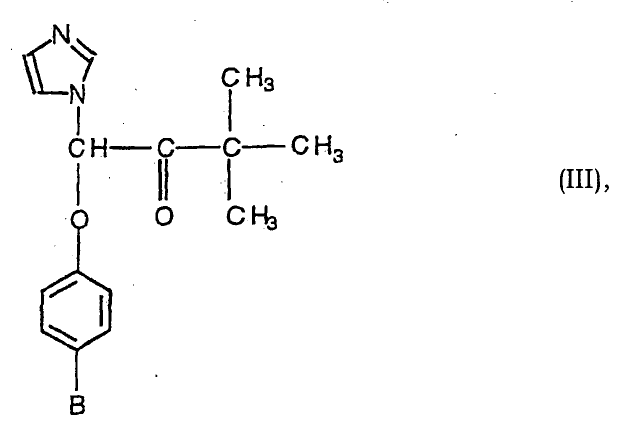 worin B ein Halogenatom bedeutet;
• oder der folgenden Formel (IV); 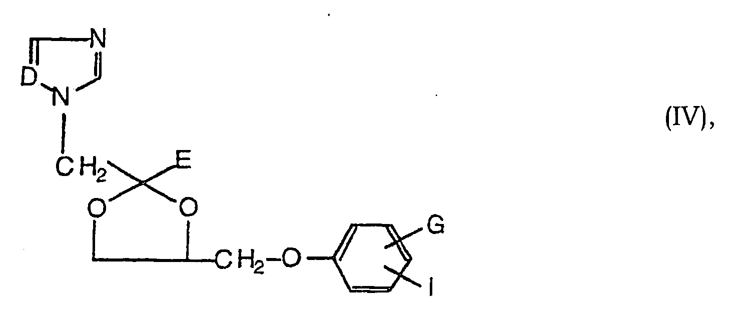 worin bedeuten:
D CH oder N;
E eine Phenylgruppe, die mit 1 bis 3 Halogenatomen, Alkyl- oder Alkoxygruppen substituiert sein kann;
G bedeutet:
a) eine Gruppe -N=C=S; oder
b) eine Gruppe der Formel 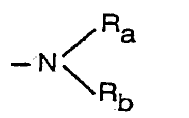 wobei Rₐ und R_{b} unabhängig voneinander ein Wasserstoffatom oder eine Alkylgruppe bedeuten; oder
c) eine Gruppe der Formel: 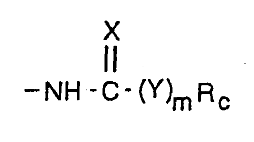 worin X ein Atom O oder S gedeutet, Y O oder NH bezeichnet, m eine ganze Zahl 0 oder 1 ist und R_{c} Wasserstoff, eine Alkylgruppe, eine mono- oder dihalogenierte Alkylgruppe, eine Phenylgruppe, die mit einem oder zwei Halogenatomen, Alkyl- oder Alkoxygruppen substituiert sein kann; oder
d) eine Gruppe der folgenden Formel 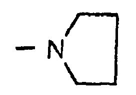 oder 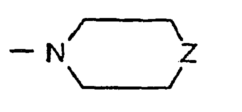
worin Z CH₂, O oder N-R₁₂ bedeutet, wobei R₁₂ ein Wasserstoffatom, eine Alkylgruppe, eine Mono- oder Polyhydroxyalkylgruppe, eine Alkoxygruppe, eine Alkoxyalkylgruppe, eine Acylgruppe, eine Alkylsulfonylgruppe, eine Phenylmethylsulfonylgruppe, eine Alkoxycarbonylgruppe, eine Acyloxygruppe, eine Alkoxycarbonylalkylgruppe, eine Phenoxycarbonylgruppe, eine Aminocarbonylgruppe, eine Mono- oder Dialkylaminocarbonylgruppe, eine Aminocarbonylalkylgruppe, eine Alkylaminocarbonylalkylgruppe, eine Alkylaminothioxoalkylgruppe, eine Alkylthioxoalkylgruppe, eine Arylgruppe, eine Arylalkylgruppe oder eine Benzoylgruppe bedeutet, die mit 1 oder 2 Halogenatomen, einer niederen Alkylgruppe oder einer niederen Alkoxygruppe substituiert sein kann; und I Wasserstoff oder eine Nitrogruppe,
oder eines ihrer Additionssalze mit physiologisch akzeptablen Säuren;
in einer kosmetischen oder dermatologischen Zusammensetzung mit einem pH-Wert von 7 oder darüber, die für die Antischuppenbehandlung der Haare und der Kopfhaut vorgesehen ist, oder für die Herstellung einer solchen Zusammensetzung, wobei die Kombination von Chlorhexidin als kationische antibakterielle Verbindung und Ketoconazol als halogenierte antimykotische Verbindung ausgenommen ist.

2. Verwendung nach Anspruch 1, **dadurch gekennzeichnet, dass** die Verbindung der Formel (I) Benzalkoniumchlorid oder Benzethoniumchlorid entspricht.

3. Verwendung nach Anspruch 1, **dadurch gekennzeichnet, dass** die Verbindung der Formel (II) dem Chlorhexidin in Salzform entspricht.

4. Verwendung nach einem der Ansprüche 1 bis 3, **dadurch gekennzeichnet, dass** die Verbindung der Formel (III) dem Climbazol entspricht.

5. Verwendung nach einem der Ansprüche 1 bis 3, **dadurch gekennzeichnet, dass** die Verbindung der Formel (IV) ausgewählt ist unter:
1-Acetyl-4-{4-[2-(2,4-dichlorphenyl)-2-(1H-imidazol-1-ylmethyl)-1,3-dioxolan-4-ylmethoxy]phenyl}-piperazin;
4-{4-[2-(2,4-Dichlorphenyl)-2-(1H-imidazol-1-ylmethyl)-1,3-dioxolan-4-ylmethoxy]phenyl}-morpholin;
N-{4-[2-(2,4-Dichlorphenyl)-2-(1H-imidazol-1-ylmethyl)-1,3-dioxolan-4-ylmethoxy]phenyl}-formamid;
4-{4-[2-(2,4-Dichlorphenyl)-2-(1H-imidazol-1-ylmethyl)-1,3-dioxolan-4-ylmethoxy]phenyl}-N-methyl-1-piperazincarboxamid;
Ethyl-4-{4-[2-(2,4-dichlorphenyl)-2-(1H-imidazol-1-ylmethyl)-1,3-dioxolan-4-ylmethoxy]phenyl}-1-piperazincarboxylat;
Methyl-4-{4-[2-(2,4-dichlorphenyl-2-(1H-imidazol-1-ylmethyl)-1,3-dioxolan-4-ylmethoxy]phenyl}-1-piperazincarboxylat;
cis-1-Acetyl-4-{4-[2-(2,4-dichlorphenyl)-2-(1H-imidazol-1-yl-methyl)-1,3-dioxolan-4-ylmethoxy]-phenyl}-piperazin;
cis-1{4-[2-(2,4-Dichlorphenyl)-2-1H-1,2,4-triazol-1-methyl)-1,3-dioxolan-4-ylmethoxy}-phenyl}-4-(1-methylethyl)-piperazin;
und deren Additionssalzen mit physiologisch akzeptablen Säuren.

6. Verwendung nach Anspruch 5, **dadurch gekennzeichnet, dass** die Verbindung der Formel (IV) das 1-Acetyl-4-{4-[2-(2,4-dichlorphenyl)-2-(1H-imidazol-1-ylmethyl)-1,3-dioxolan-4-ylmethoxy]phenyl}-piperazin oder eines seiner Additionssalze mit physiologisch akzeptablen Säuren ist.

7. Verwendung nach einem der Ansprüche 1 bis 6, **dadurch gekennzeichnet, dass** die Additionssalze mit physiologisch akzeptablen Säuren unter Salzen von Schwefelsäure, Salpetersäure, Thiocyansäure, Salzsäure, Bromwasserstoffsäure, Iodwasserstoffsäure, Phosphorsäure, Essigsäure, Benzoesäure, Salicylsäure, Glycolsäure, Acetursäure, Bernsteinsäure, Nicotinsäure, Weinsäure, Maleinsäure, Palmitinsäure, Methansulfonsäure, Propionsäure, Hydroxyessigsäure, 2-Hydroxypropansäure, 2-Oxopropansäure, Ethandisäure, Propandisäure, 1,4-Butandisäure, 2-Hydroxy-1,4-butandisäure, 2,3-Dihydroxy-1,4-butandisäure, 2-Hydroxy-1,2,3-propantricarbonsäure, 3-Phenyl-2-propensäure, α-Hydroxybenzolessigsäure, Ethansulfonsäure, 2-Hydroxyethansulfonsäure, 4-Methylbenzolsulfonsäure, 2-Hydroxybenzoesäure, 4-Amino-2-hydroxybenzoesäure, 2-Phenoxybenzoesäure, 2-Acetyloxybenzoesäure, Pikrinsäure, Milchsäure und Aminosäuren ausgewählt sind.

8. Verwendung nach einem der Ansprüche 1 bis 7, **dadurch gekennzeichnet, dass** die Verbindung (a) und die Verbindung (b) in Mengenanteilen von 0,001 bis 10 Gew.-%, bezogen auf das Gesamtgewicht der Zusammensetzung, enthalten sind.

9. Verwendung nach einem der Ansprüche 1 bis 8, **dadurch gekennzeichnet, dass** die Verbindung (a) und die Verbindung (b) in Mengenanteilen von 0,01 bis 5 Gew.-%, bezogen auf das Gesamtgewicht der Zusammensetzung, enthalten sind.

10. Verwendung nach einem der Ansprüche 1 bis 9, **dadurch gekennzeichnet, dass** das Gewichtsverhältnis von Verbindung (a) und Verbindung (b) im Bereich von 0,1 bis 10 liegt.

11. Verwendung nach Anspruch 10, **dadurch gekennzeichnet, dass** das Gewichtsverhältnis von Verbindung (a) und Verbindung (b) im Bereich von 0,3 bis 3 liegt.

12. Verwendung nach einem der Ansprüche 1 bis 11, **dadurch gekennzeichnet, dass** der pH-Wert der Zusammensetzung im Bereich von 7,2 bis 9 und vorzugsweise 7,5 bis 8,5 liegt.

13. Verwendung nach einem der Ansprüche 1 bis 12, **dadurch gekennzeichnet, dass** die Zusammensetzung als mehr oder weniger dickflüssige Flüssigkeit, Gel, Creme oder Aerosolschaum vorliegt.

14. Verwendung nach einem der Ansprüche 1 bis 13, **dadurch gekennzeichnet, dass** die Zusammensetzung mindestens einen anionischen, amphoteren, zwitterionischen oder kationischen grenzflächenaktiven Stoff enthält.

15. Verwendung nach einem der Ansprüche 1 bis 14, **dadurch gekennzeichnet, dass** die Zusammensetzung mindestens einen nichtionischen grenzflächenaktiven Stoff enthält.

16. Verwendung nach Anspruch 15, **dadurch gekennzeichnet, dass** die Zusammensetzung mindestens einen nichtionischen grenzflächenaktiven Stoff vom mehrfach mit Glycerin veretherten Typ oder Alkylpolyglycosidtyp enthält.

17. Verwendung nach Anspruch 14 oder 16, **dadurch gekennzeichnet, dass** der nichtionische grenzflächenaktive Stoff in Konzentrationen von 5 bis 50 Gew.-%, bezogen auf das Gesamtgewicht der Zusammensetzung, enthalten ist.

18. Verwendung nach einem der Ansprüche 1 bis 17, **dadurch gekennzeichnet, dass** die Zusammensetzung Zusatzstoffe enthält, die unter den Suspendiermitteln, anionischen, kationischen, nichtionischen, amphoteren Polymeren, Proteinen, Ölen, Wachsen, Silicongummis und/oder Siliconharzen, Ansäuerungsmitteln oder Alkalisierungsmitteln, Konservierungsmitteln, antibakteriellen Wirkstoffen und antimykotischen Wirkstoffen, die von den in Anspruch 1 definierten Verbindungen verschieden sind, Parfums und anderen, gewöhnlich in der Kosmetik verwendeten Zusatzstoffen ausgewählt sind.

19. Verwendung nach einem der Ansprüche 1 bis 18, **dadurch gekennzeichnet, dass** die Zusammensetzung als Haarwaschmittel zum Waschen der Haare und zur Behandlung der Kopfhaut vorliegt.

20. Verfahren zur kosmetischen Behandlung, um Schuppen der Haare und der Kopfhaut zu entfernen, **dadurch gekennzeichnet, dass** eine Zusammensetzung, wie sie in einem der Ansprüche 1 bis 19 definiert ist, aufgetragen wird und anschließend gegebenenfalls gespült wird.
